(19) Europäisches Patentamt
European Patent Office
Office européen des brevets

(11) Veröffentlichungsnummer: **0 004 279**
**B1**

# (12) EUROPÄISCHE PATENTSCHRIFT

(45) Veröffentlichungstag der Patentschrift: **29.12.82**

(21) Anmeldenummer: **79100423.7**

(22) Anmeldetag: **13.02.79**

(51) Int. Cl.³: **C 07 D 471/04,** C 07 D 215/56, C 07 D 413/04, A 61 K 31/47, A 61 K 31/505 // A23K1/00, (C07D471/04, 221/00, 221/00),(C07D471/04, 221/00, 239/00)

(54) **Verfahren zur Herstellung von 4-Pyridon-3-carbonsäuren, 1-Cyclopropyl-4-Pyridon-3-Carbonsäurederivate und diese enthaltende Arzneimittel.**

(30) Priorität: **24.02.78 DE 2808070**

(43) Veröffentlichungstag der Anmeldung: **03.10.79 Patentblatt 79/20**

(45) Bekanntmachung des Hinweises auf die Patenterteilung: **29.12.82 Patentblatt 82/52**

(84) Benannte Vertragsstaaten: **BE CH DE FR GB IT LU NL SE**

(56) Entgegenhaltungen:
**DE-A-1 670 533**
**DE-A-2 246 503**
**DE-A-2 338 325**
**DE-A-2 360 329**
**DE-A-2 421 121**
**FR-A-2 348 213**
**US-A-3 673 184**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Grohe, Klaus, Dr., Am Wasserturm 10, D-5068 Odenthal (DE)**
Erfinder: **Zeiler, Hans-Joachim, Dr., Windrather Strasse 188, D-5620 Velbert 15 (DE)**
Erfinder: **Metzger, Karl Georg, Dr., Pahlkestrasse 15, D-5600 Wuppertal 1 (DE)**

EP 0 004 279 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von 4-Pyridon-3-carbonsäuren, 1-Cyclopropyl-4-Pyridon-3-Carbonsäurederivate und diese enthaltende Arzneimittel

Die Erfindung betrifft ein Verfahren zur Herstellung von 4-Pyridon-3-carbonsäuren und/oder deren Derivaten, neue 1-Cyclopropyl-4-Pyridon-3-carbonsäuren und/oder deren Derivate, sowie ihre Verwendung als Arzneimittel, insbesondere als antibakterielle Mittel, sowie als Futterzusatzmittel.

In J. Het. Chem. 12, 557 (1975) wird die Herstellung von 1-Äthyl-4-chinolon-3-carbonsäureäthylester durch Umsetzung von N-Äthyl-anilin mit Äthoxy-methylen-malonsäurediäthylester und anschließende Cyclisierung in Gegenwart von Polyphosphorsäureester bei erhöhter Temperatur beschrieben.

Gegenstand der Erfindung ist ein Verfahren zur Herstellung von 4-Pyridon-3-carbonsäuren und/oder deren Derivaten, das dadurch gekennzeichnet ist, daß man Enamine der Formel

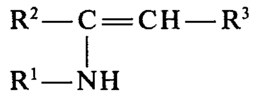

(I)

in der

$R^1$ gesättigtes oder ungesättigtes, geradkettiges oder verzweigtes Alkyl mit 1—6 Kohlenstoffatomen, gesättigtes oder ungesättigtes, gegebenenfalls durch Methyl- oder Äthylgruppen substituiertes Cycloalkyl mit 3—7 Kohlenstoffatomen, Aralkyl mit 1—4 Kohlenstoffatomen im aliphatischen Teil und 6—10 Kohlenstoffatomen im aromatischen Teil, Aryl mit 6—10 Kohlenstoffatomen oder eine Aminogruppe $-NR^4R^5$ bedeutet, in der $R^4$ und $R^5$ gleich oder verschieden sein können und für geradkettiges oder verzweigtes $C_1$—$C_4$-Alkyl stehen oder gemeinsam mit dem Stickstoffatom, das sie substituieren und gegebenenfalls einem weiteren Heteroatom einen 5- bis 7gliedrigen Ring bilden können,

$R^2$ Wasserstoff, gesättigtes oder ungesättigtes, geradkettiges oder verzweigtes Alkyl mit 1—6 Kohlenstoffatomen, Aralkyl mit 1—4 Kohlenstoffatomen im aliphatischen Teil und 6—10 Kohlenstoffatomen im aromatischen Teil oder Aryl mit 6—10 Kohlenstoffatomen bedeutet und

$R^3$ eine Nitrilgruppe, eine Estergruppe $-COOR^6$ oder eine Säureamidgruppe $-CONR^7R^8$, wobei $R^6$, $R^7$ und $R^8$ für Wasserstoff oder $C_1$—$C_4$-Alkyl stehen, $R^8$ außerdem gegebenenfalls substituiertes Phenyl sein kann, bedeutet,

mit o-Halogen-(hetero)aryl-carbonsäurehalogeniden der Formel

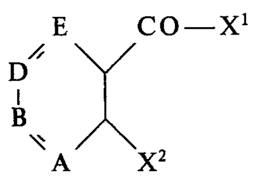

(II)

in der bis zu 3 der Symbole A, B, D und E ein Stickstoffatom sein können und die jeweils restlichen der Symbole A, B, D und E ein gegebenenfalls durch $C_1$—$C_6$-Alkyl, $C_1$—$C_4$-Alkoxy, $C_1$—$C_6$-Alkylmerkapto, Trifluormethyl, Halogen, Cyano, durch $C_1$—$C_4$-Alkyl verestertes Carboxyl, Benzyl oder Phenyl, die jeweils durch $C_1$—$C_3$-Alkyl, Nitro oder Halogen substituiert sein können, oder durch Carbalkoxy substituiertes Amino substituiertes Kohlenstoffatom bedeuten, und

$X^1$ und $X^2$ gleiches oder verschiedenes Halogen darstellen,

in einer ersten Reaktionsstufe in einem wasserfreien, aprotischen Lösungsmittel in Gegenwart einer Base bei 0 bis 80°C und in einer zweiten Reaktionsstufe in Gegenwart einer Base bei 80 bis 250°C umsetzt, gegebenenfalls die Gruppe $R^3$ in an sich bekannter Weise in die Carboxylgruppe und gegebenenfalls diese in ihre Salze überführt.

Es ist als ausgesprochen überraschend zu bezeichnen, daß die Enamine der Formel (I) durch die o-Halogen-(hetero) aroyl-halogenide der Formel (II) in der ersten Reaktionsstufe des erfindungsgemäßen Verfahrens an dem zum Stickstoff in $\beta$-Stellung befindlichen Kohlenstoff acyliert werden, während aus Chem. Ber. 50, 65 (1917) bekannt ist, daß Benzoylchloride, die in ortho-Stellung durch Brom, Nitro oder Acetoxy substituiert sind, nur unter Stickstoff-Acylierung reagieren. Weiterhin ist es überraschend, daß die Zwischenprodukte der ersten Reaktionsstufe des erfindungsgemäßen Verfahrens der Formel (VI) trotz der durch die Doppelbildung möglichen cis-trans-Isomerie in eindeutiger Weise zum erfindungsgemäßen Endprodukt der Formel (VII) weiterreagieren.

Der Vorteil des erfindungsgemäßen Verfahrens ist es, daß in einer einfach durchzuführenden, sogenannten Eintopf-Reaktion, nur einheitliche Verbindungen entstehen.

Selbstverständlich ist es möglich, aus den erfindungsgemäß erhaltenen 4-Pyridon-3-carbonsäurederivaten durch geeignete und dem Fachmann bekannte saure oder alkalische Verseifungsmethoden die freien Carbonsäuren und nachfolgend die pharmazeutisch anwendbaren Carbonsäuresalze, beispielsweise die Alkali- oder Erdalkalisalze, herzustellen.

Als Alkyl ($R^1$, $R^2$) kommen gesättigte oder ungesättigte, geradkettige oder verzweigte Alkylreste mit 1 bis 6 Kohlenstoffatomen in Betracht, beispielsweise Methyl, Äthyl, Propyl, Isopropyl, Butyl, Isobutyl, tert.-Butyl, Pentyl, Hexyl, Propenyl, Butenyl, Pentenyl oder Hexenyl. Bevorzugte aliphatische Reste ($R^1$, $R^2$) sind solche mit 1 bis 4 Kohlenstoffatomen, beispielsweise Methyl, Äthyl, Propyl, Isopropyl, Butyl, Isobutyl oder tert.-Butyl.

Besonders bevorzugte aliphatische Reste ($R^1$) sind Äthyl oder tert.-Butyl.

Als Cycloalkyl ($R^1$) kommen gesättigte oder ungesättigte, gegebenenfalls durch Methyl- oder Äthylgruppen substituierte carbocyclische Ringsysteme mit 3 bis 7 Kohlenstoffatomen in Betracht, beispielsweise Cyclopropyl, Cyclopropenyl, Cyclobutyl, Cyclobutenyl, Cyclopentyl, Cyclopentenyl, Cyclohexyl, Cyclohexenyl, Cycloheptyl oder Cycloheptenyl. Als bevorzugte cycloaliphatische Reste ($R^1$) seien der Cyclopropylrest und der Cyclohexylrest genannt.

Als Aralkyl ($R^1$, $R^2$) seien Reste mit 1 bis 4 Kohlenstoffatomen im aliphatischen Teil und 6 bis 10 Kohlenstoffatomen im aromatischen Teil genannt, beispielsweise Benzyl, $\beta$-Phenyläthyl, Naphthylmethyl oder $\beta$-Naphthyläthyl. Bevorzugt ist der Benzylrest.

Als Aryl ($R^1$, $R^2$) seien aromatische, carbocyclische Ringsysteme mit 6 bis 10 Kohlenstoffatomen genannt, beispielsweise Phenyl, Naphthyl, o-Biphenyl, m-Biphenyl oder p-Biphenyl. Bevorzugt ist der Phenylrest.

Als Aminogruppe ($R^1$) kommt die Gruppierung $-NR^4R^5$ in Betracht. Hierin bedeuten $R^4$ und $R^5$ gleiche oder verschiedene, geradkettige oder verzweigte $C_1-C_4$-Alkylreste, beispielsweise Methyl, Äthyl, Propyl, Isopropyl, Butyl, oder Isobutyl, bevorzugt Methyl und Äthyl, besonders bevorzugt Methyl.

Die Alkylreste ($R^4$, $R^5$) können auch gemeinsam mit dem Stickstoffatom der Aminogruppe, das sie substituieren, und gegebenenfalls einem weiteren Heteroatom einen 5- bis 7gliedrigen heterocyclischen Ring bilden. Als gegebenenfalls weitere Heteroatome kommen Sauerstoff, Schwefel oder Stickstoff, bevorzugt Sauerstoff oder Schwefel in Betracht.

Als solche heterocyclische Ringsysteme seien beispielsweise genannt: Pyrrolidin, Piperidin, Hexamethylenimin, Morpholin oder Thiomorpholin, bevorzugt Morpholin.

Die Reste $R^7$ und $R^8$ können außerdem für Wasserstoff stehen. Der Rest $R^8$ kann weiterhin für gegebenenfalls substituiertes Phenyl stehen.

Die Symbole A, B, D und E bilden gemeinsam einen an der 5- und 6-Stellung des Pyridonringes annellierten carbocyclischen oder heterocyclischen aromatischen Ring. Als Heteroatome kommen bis zu 3 Stickstoffatome in Betracht.

Die genannten Symbole A bis E können beispielsweise folgende Bedeutung annehmen:
Alle Symbole A bis E sind gegebenenfalls substituierte Kohlenstoffatome;

A ist ein Stickstoffatom und B, D und E sind gegebenenfalls substituierte Kohlenstoffatome;
D ist ein Stickstoffatom und A, B und E sind gegebenenfalls substituierte Kohlenstoffatome;
A, B sind Stickstoffatome und D, E sind gegebenenfalls substituierte Kohlenstoffatome;
A, D sind Stickstoffatome und B, E sind gegebenenfalls substituierte Kohlenstoffatome;
A, E sind Stickstoffatome und B, D sind gegebenenfalls substituierte Kohlenstoffatome;
A, B, E sind Stickstoffatome und D ist ein gegebenenfalls substituiertes Kohlenstoffatom;
A, D, E sind Stickstoffatome und B ist ein gegebenenfalls substituiertes Kohlenstoffatom.

Als gegebenenfalls substituierte Kohlenstoffatome kommt die Gruppierung $-C-R^9$ in Betracht, worin $R^9$ bei den einzelnen Kohlenstoffatomen gleich oder verschieden sein kann und beispielsweise folgende Bedeutung haben kann: Wasserstoff, $C_1-C_6$-Alkyl, $C_1-C_4$-Alkoxy, $C_1-C_6$-Alkylmercapto, Trifluormethyl, Halogen, Cyano, durch $C_1-C_4$-Alkyl verestertes Carboxyl, Benzyl oder Phenyl, die jeweils durch $C_1-C_3$-Alkyl, Nitro oder Halogen substituiert sein können, oder durch carbalkoxysubstituiertes Amino. Als Halogen sei beispielsweise genannt: Fluor, Chlor, Brom, Jod, bevorzugt Chlor oder Brom.

Zwei der Reste $R^9$, die sich an zwei benachbarten Kohlenstoffatomen, beispielsweise an A und B oder an B und D oder an D und E befinden, können gemeinsam mit den beiden benachbarten Kohlenstoffatomen einen weiteren annellierten Benzolkern darstellen.

Selbstverständlich können alle aufgezählten Alkyl-, Cycloalkyl-, Aralkyl- oder Arylreste ($R^1$ bis $R^8$) durch Substituenten substituiert sein, die unter den Reaktionsbedingungen des erfindungsgemäßen Verfahrens inert sind. Als solche kommen beispielsweise die für die Definition von $R^9$ genannten Substituenten in Betracht.

Halogen ($X^1$, $X^2$) können Halogenatome, beispielsweise Fluor, Chlor, Brom oder Jod, bevorzugt Chlor oder Brom, besonders bevorzugt Chlor, sein.

Das erfindungsgemäße Verfahren wird bevorzugt durch den Einsatz von Enaminen der Formel

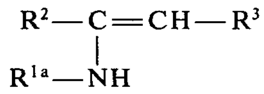

(III)

in der

$R^{1a}$ tert.-Alkyl, $C_3-C_7$-Cycloalkyl, die Dialkylamino-Gruppe $-NR^4R^5$,
worin $R^4$ und $R^5$ geradkettiges oder verzweigtes $C_1-C_2$-Alkyl bedeutet,
oder Morpholinyl darstellt,
$R^2$ für Wasserstoff, $C_1-C_4$-Alkyl oder gegebenenfalls substituiertes Benzyl oder Phenyl steht und
$R^3$ die oben angegebene Bedeutung besitzt,

durchgeführt.

Das erfindungsgemäße Verfahren wird besonders bevorzugt durch den Einsatz von Enaminen der Formel

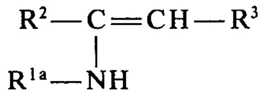

(IV)

in der

$R^{1a}$ für tert.-Butyl, Cyclopropyl, Cyclohexyl oder die Dimethylamino-Gruppe oder N-Morpholinyl darstellt
$R^2$ Wasserstoff oder $C_1-C_4$-Alkyl bedeutet und
$R^3$ die obengenannte Bedeutung besitzt,

durchgeführt.

Enamine, die im erfindungsgemäßen Verfahren eingesetzt werden können, können beispielsweise nach Chem. Ber. 99, 2526 (1966) durch Umsetzung von Propiolsäure-Methylester und primären Aminen oder nach Chem. Ber. 108, 1659 (1975) aus N,N-disubstituierten Hydrazinen mit Acetessigsäure-Derivaten hergestellt werden.

Beispielsweise seien die folgenden $\beta$-Enamino- und $\beta$-Enhydrazino-Carbonsäurederivate genannt:

$\beta$-Methylaminocrotonsäuremethylester, $\beta$-Äthylaminocrotonsäuremethylester,
$\beta$-Äthylaminocrotonsäureäthylester, $\beta$-Äthylaminocrotonsäure-n-propylester,
$\beta$-n-Propylaminocrotonsäuremethylester, $\beta$-Cyclopentylaminocrotonsäuremethylester,
$\beta$-Cyclohexylaminocrotonsäureäthylester,
3-(2,2-Dimethylhydrazino)-crotonsäuremethylester,
3-(2,2-Dimethylhydrazino)-crotonsäureäthylester,
$\beta$-Morpholinyl-aminocrotonsäuremethylester,
$\beta$-Piperidinylamino-crotonsäureäthylester, $\beta$-Äthylamino-acrylsäuremethylester,
$\beta$-i-Propylamino-acrylsäuremethylester,
$\beta$-Cyclopropylamino-acrylsäuremethylester,
$\beta$-Cyclohexylamino-acrylsäureäthylester,
$\beta$-t-Butylamino-acrylsäuremethylester,
$\beta$-Morpholinylaminoacrylsäureäthylester, $\beta$-Methylaminocrotonsäureanilid,
$\beta$-Morpholinyl-amino-crotonsäure-(4-chloranilid),
$\beta$-Methylaminocrotonsäurenitril, $\beta$-Anilino-crotonsäuremethylester,
$\beta$-Anilino-acrylsäuremethylester, $\beta$-Anilino-acrylsäureäthylester,
$\beta$-(4-Methoxy-phenylamino)-crotonsäureäthylester.

Das erfindungsgemäße Verfahren wird bevorzugt durch den Einsatz solcher o-Chlor-(hetero-aryl-carbonsäurechloride der Formel

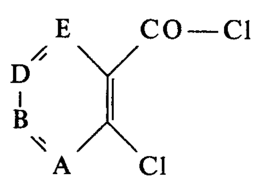

(V)

in der
die Symbole A, B, D und E die obengenannte Bedeutung haben, der gegebenenfalls vorhandene Heterocyclus jedoch höchstens zwei Stickstoffatome enthält.

Als gegebenenfalls substituierte Kohlenstoffatome kommt jeweils die weiter oben beschriebene Gruppe $-CR^9$ in Betracht.

o-Halogen-(hetero)aryl-carbonsäurehalogenide, die im erfindungsgemäßen Verfahren eingesetzt werden können, und Verfahren zu ihrer Herstellung sind bekannt. So beschreibt beispielsweise J. Am. Chem. Soc. 40, 233 (1908) die Herstellung von 2-Äthylmercapto-6-chlorpyrimidin-5-carbonsäurechlorid durch Umsetzung von 2-Äthylmercapto-4-oxo-3,4-dihydropyrimidin-5-carbonsäure und Phosphoroxychlorid. In Arch. Pharm. 306, 401 (1973) wird die Umsetzung von 2-Hydroxy-chinoxalin-3-carbonsäure mit Thionylchlorid in Gegenwart einer katalytischen Menge Dimethylformamid zu 2-Chlor-chinoxalin-3-carbonsäurechlorid beschrieben. In J. Chem. Soc. (C), 1966, 1816 wird die Umsetzung von 4-Chlornicotinsäure mit Thionylchlorid unter Rückfluß zu 4-Chlornicotinsäurechlorid beschrieben.

Als o-Halogen-(hetero)aryl-carbonsäurehalogenide, die im erfindungsgemäßen Verfahren eingesetzt werden können, seien beispielsweise genannt:

2-Chlor-5-nitro-benzoylchlorid, 2-Chlor-5-nitro-benzoylbromid,
2-Chlor-3-nitro-benzoylchlorid,
2-Chlor-3-nitro-5-trifluormethyl-benzoylchlorid,
2,4-Dichlor-3-nitro-benzoylchlorid, 2,4-Dichlor-5-nitro-benzoylchlorid,
2,4-Dichlor-3,5-dinitro-benzoylchlorid, 2,6-Dichlor-3,5-dinitro-benzoylchlorid,
2-Chlor-3,5-Dinitro-benzoylchlorid, 2-Chlor-3,5-dinitro-benzoylbromid,
2,4,5-Trichlor-3-nitro-benzoylchlorid, 2,4,6-Trichlor-3,5-dinitro-benzoylchlorid,
2-Fluor-5-nitro-benzoylchlorid, 2-Chlor-nicotinsäurechlorid,
2-Chlor-4-methyl-nicotinsäurechlorid, 2-Chlor-6-methyl-nicotinsäurechlorid,
4-Chlornicotinsäurechlorid, 2,6-Dichlor-nicotinsäurechlorid,
2,5,6-Trichlor-nicotinsäurechlorid, 2-Brom-nicotinsäurechlorid,
2,5-Dichlor-nicotinsäurechlorid, 2-Chlor-5-nitro-nicotinsäurechlorid,
2-Chlor-4,6-Dimethyl-5-nitro-nicotinsäurechlorid,
2-Methylmercapto-4-chlor-pyrimidin-5-carbonsäurechlorid,
2-Äthylmercapto-4-chlor-pyrimidin-5-carbonsäurechlorid,
4-Chlor-6-methoxy-pyrimidin-5-carbonsäurechlorid,
4-Chlor-6-phenyl-pyrimidin-5-carbonsäurechlorid,
2,4-Dichlor-pyrimidin-5-carbonsäurechlorid,
3-Chlor-pyridazin-4-carbonsäurechlorid,
2,6-Dichlor-pyridazin-4-carbonsäurechlorid,
2-Chlor-pyrazin-3-carbonsäurechlorid, 2-Chlor-chinoxalin-3-carbonsäurechlorid.

Das erfindungsgemäße Verfahren wird in der ersten Reaktionsstufe im Temperaturbereich von etwa 0 bis 80°C und in der zweiten Reaktionsstufe im Temperaturbereich von etwa 80 bis 250°C durchgeführt. Bevorzugt wird in der ersten Reaktionsstufe bei etwa 10 bis 60°C und in der zweiten Reaktionsstufe bei etwa 100 bis 150°C gearbeitet.

Das erfindungsgemäße Verfahren wird in einem wasserfreien, aprotischen Lösungsmittel durchgeführt. Ein solches Lösungsmittel kann beispielsweise der Gruppe der Kohlenwasserstoffe, wie etwa Ligroin, Cyclohexan, Benzol oder Toluol, der Gruppe der chlorierten Kohlenwasserstoffe, wie etwa Methylenchlorid, Chloroform, Tetrachlorkohlenstoff, Chlorbenzol oder o-Dichlorbenzol, der Gruppe der Nitrile, wie etwa Acetonitril, oder der Gruppe der Äther, wie etwa Diäthyläther, Tetrahydrofuran oder Dioxan angehören. Bevorzugt ist der Einsatz von Dioxan als Lösungsmittel.

Die über etwa 80°C siedenden Lösungsmittel aus der beispielhaften Aufzählung können auch direkt für die zweite Reaktionsstufe weiterverwendet werden. Bei dieser Verfahrensvariante können beide Reaktionsstufen in einer sogenannten Eintopf-Reaktion durchgeführt werden.

In einer anderen Verfahrensvariante wird nach Durchführung der ersten Reaktionsstufe das niedrig siedende Lösungsmittel abdestilliert und durch ein höher siedendes Lösungsmittel ersetzt. In diesem Fall können außer den beispielhaft aufgezählten höhersiedenden Lösungsmitteln noch weitere Lösungsmittel in Betracht kommen, beispielsweise Dimethylformamid, Dimethylsulfoxid, N-Methylpyrrolidon, Sulfolan oder Hexamethylphosphorsäuretriamid.

In einer weiteren Verfahrensvariante kann mit dem niedrig siedenden Lösungsmittel aus der ersten Reaktionsstufe weitergearbeitet werden, wenn die zweite Reaktionsstufe unter Druck durchgeführt wird. Üblicherweise wird das erfindungsgemäße Verfahren jedoch bei Normaldruck durchgeführt.

Das erfindungsgemäße Verfahren wird in beiden Reaktionsstufen in Gegenwart von Basen durchgeführt. Als Basen für die erste Reaktionsstufe des erfindungsgemäßen Verfahrens seien tertiäre organische Amine genannt, beispielsweise Pyridin, Triäthylamin, N-Methylmorpholin, N-Methylpiperidin, Chinolin oder Triäthylendiamin. Bevorzugt wird in Gegenwart von Pyridin oder Triäthylamin gearbeitet.

Die zweite Reaktionsstufe des erfindungsgemäßen Verfahrens wird in Gegenwart von zusätzlich zugefügter Base durchgeführt. Hierfür kommen beispielsweise in Betracht: 1,5-Diazabicyclo-[4.3.0]-non-5-en(DBN), 1,8-Diazabicyclo-[5.4.0]-undec-7-en(DBU), Triäthylendiamin, Triäthylamin, Kaliumhydroxid, Natriumhydroxid, Natriummethylat, Natriumäthylat, Natriumhydrid, Butyl-lithium, Lithiumphenyl oder Phenylmagnesiumbromid. Für die zweite Reaktionsstufe wird der Einsatz von 1,8-Diazabicyclo-[5.4.0]-undec-7-en(DBU) bevorzugt.

Die Ausgangsverbindungen des erfindungsgemäßen Verfahrens der Formel (I) und (II) sowie die Base im ersten Reaktionsschritt werden im allgemeinen in äquimolaren Mengen zueinander eingesetzt. In der zweiten Reaktionsstufe wird eine weitere äquimolare Menge Base zugesetzt. Es kann vorteilhaft sein, einen Überschuß von 10 Mol-% an Base einzusetzen.

Das erfindungsgemäße Verfahren kann am Beispiel der Umsetzung von 2-Äthylmercapto-4-chlor-pyrimidin-5-carbonsäurechlorid mit $\beta$-Methylamino-crotonsäureäthylester durch die folgenden Formelgleichungen dargestellt werden:

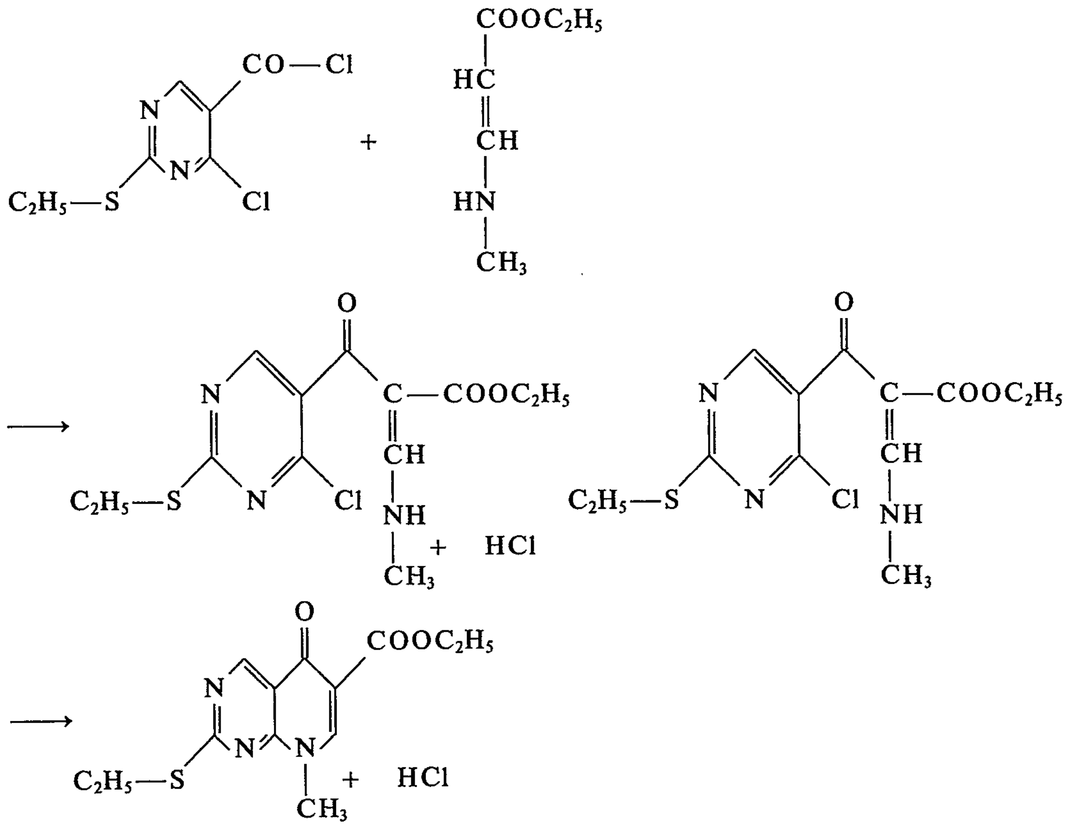

Das erfindungsgemäße Verfahren kann beispielsweise wie folgt durchgeführt werden:

a) eine Lösung des o-Halogen-(hetero)aroyl-halogenids der Formel (II) in einem wasserfreien, aprotischen Lösungsmittel wird nacheinander mit dem Enamin der Formel (I) und dem tertiären Amin aus der Gruppe der Basen für die erste Reaktionsstufe versetzt. Nach Beendigung der Reaktion wird das Lösungsmittel abdestilliert. Zur Aufarbeitung kann man das Reaktionsgemisch beispielsweise in einem Gemisch aus einem wasserunlöslichen Lösungsmittel, wie etwa Methylenchlorid oder Chloroform, und Wasser aufnehmen und waschen. Aus der organischen Phase wird durch Abdestillieren des Lösungsmittels das Zwischenprodukt der Formel (VI)

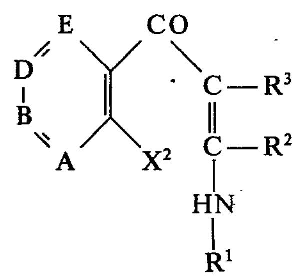

(VI)

erhalten, das durch Umkristallisation gereinigt werden kann. Das Reaktionsprodukt der ersten Reaktionsstufe der Formel (VI) wird dann in einem wasserfreien, aprotischen Lösungsmittel mit einem Siedepunkt oberhalb von 80° C mit der äquimolaren Menge einer Base aus der Gruppe für die zweite Reaktionsstufe weiter umgesetzt. Die Aufarbeitung erfolgt in der analogen Weise wie im Anschluß an die erste Reaktionsstufe. Zur Reinigung kann beispielsweise aus Äthanol, Acetonitril oder Essigester umkristallisiert werden.

b) Die Reihenfolge der Zugabe der Ausgangsmaterialien für die erste Reaktionsstufe kann geändert werden. So kann die Reihenfolge beispielsweise lauten:

1. Enamin,
2. o-Halogen-(hetero)aroyl-halogenid,
3. die Base.

Die Reihenfolge der Zugabe kann beispielsweise ferner lauten:

1. Enamin und Base gemeinsam und
2. das o-Halogen-(hetero)aroyl-halogenid.

c) Verwendet man für die erste Reaktionsstufe ein wasserfreies, aprotisches Lösungsmittel mit einem Siedepunkt oberhalb 80°C, so können beide Reaktionsstufen des erfindungsgemäßen Verfahrens in einer Apparatur hintereinander und ohne besondere Isolierung des Zwischenprodukts der Formel (VI) in einer sogenannten Eintopf-Reaktion durchgeführt werden. Vor Erhöhung der Reaktionstemperatur für die zweite Reaktionsstufe wird lediglich eine Base aus der Gruppe der für die zweite Reaktionsstufe geeigneten zugesetzt. Da die Basen, die weiter oben für die zweite Reaktionsstufe genannt sind, beispielsweise DBU, häufig stärker sind als die Basen, die weiter oben für die erste Reaktionsstufe genannt worden sind, ist es bei der Durchführung des Eintopf-Verfahrens häufig notwendig, für die zweite Reaktionsstufe die doppelt molare Menge an Base einzusetzen, da die Hälfte hiervon zur Freisetzung der schwächeren Base der ersten Reaktionsstufe notwendig ist.

Die Erfindung betrifft weiterhin neue 1-Cyclopropyl-4-Pyridon-3-carbonsäuren und/oder deren Derivate der Formel (IX)

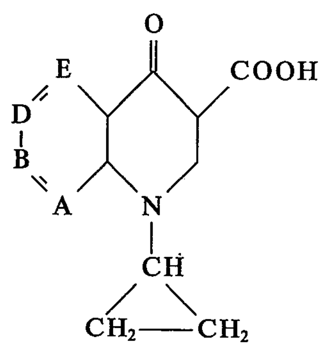

in der

A, B, D und E die obengenannte Bedeutung haben sowie deren pharmazeutisch verwendbaren Salze.

Es wurde weiterhin gefunden, daß die erfindungsgemäßen neuen Verbindungen hervorragende antibakterielle und fungizide Eigenschaften besitzen und darüber hinaus als Wachstumsregulatoren wirksam sind.

Insbesondere sind sie bakteriostatisch und bakterizid, beispielsweise gegen gramnegative Bakterien, wie Escherichia, Proteus und Klebsiella, wirksam. Die verbesserte antibakterielle Wirkung der erfindungsgemäßen neuen Verbindungen wird besonders deutlich am Beispiel der 1-Cyclopropyl-7-methyl-4-naphthyridon-(1,8)-3-carbonsäure (Verbindung aus Beispiel 19), die sich im Vergleich mit der bekannten 1-Ethyl-7-methyl-4-naphthyridon-(1,8)-3-carbonsäure (»Nalidixinsäure«; Ehrhart/Ruschig, Arzneimittel, Band 2: Chemotherapeutika, Verlag Chemie 1968, Seite 1568) in vitro und in vivo bei Straphylokokken, Escherichio coli, Proteus, Klebsiella, Pseudomonas usw. als weit überlegen erwies.

Dies geht aus der nachstehenden Tabelle hervor, in der die Wirksamkeiten der Verbindung aus Beispiel 19 mit denjenigen der Nalidixinsäure in vitro und in vivo gegenübergestellt sind.

1. Wirksamkeit in vitro

Minimale Hemmkonzentrationen in mcg/ml

Die minimalen Hemmkonzentrationen gegen Bakterien wurden im Agarverdünnungstest ermittelt. Dazu wurden verschiedene Präparatkonzentrationen zusammen mit dem Teststamm in flüssigem

DTS-Agar-Medium suspendiert und in Petrischalen (Durchmesser 5 cm) gegossen. Die Keimeinsaat pro Platte betrug $5 \times 10^3$ Keime. Als MHK wird die geringste Präparatkonzentration bezeichnet, bei der innerhalb von 24 Std. keine Koloniebildung mehr stattfand.

| Stamm | Verbindung aus Beispiel 19 | Nalidixinsäure |
|---|---|---|
| E. coli Neumann | 0,5 | 1 |
| E. coli T7 | 0,5 | 2 |
| E. coli 4339 | 0,5 | 4 |
| Klebsiella 6312 | 2 | 8 |
| Proteus morganii 11003 | 1 | 4 |
| Providencia 12004 | 1 | 4 |

2. Wirksamkeit im Tierversuch (Maus)

| Infektionskeim | Therapie p. o. 30' nach Infekt $ED_{(90-100)}$, mg/kg | |
|---|---|---|
| | Verbindung aus Beispiel 19 | Nalidixinsäure |
| E. coli Neumann | 40 | 80–160 |
| Proteus vulg. 1017 | 20–40 | 80–160 |
| Klebs. 63 | 20–40 | 160 |
| Klebs. 8085 | 40–80 | 160 |

Die verbesserte antibakterielle Wirksamkeit der erfindungsgemäßen Verbindungen ermöglicht ihren Einsatz als Wirkstoffe sowohl in der Human- als auch in der Veterinärmedizin, wobei sie sowohl zur Verhütung als auch zur Behandlung von systemischen oder lokalen bakteriellen Infektionen verwendet werden können. Die erfindungsgemäßen Verbindungen können weiterhin auch als Futterzusatzmittel zur Förderung des Wachstums und zur Verbesserung der Futterauswertung in der Tierhaltung, insbesondere bei der Haltung von Mastvieh, verwendet werden. Die Applikation der Wirkstoffe erfolgt dann vorzugsweise über das Futter und/oder das Trinkwasser.

Die vorliegende Erfindung betrifft weiterhin Mittel, die die neuen erfindungsgemäßen Verbindungen enthalten. Hierzu gehören beispielsweise Futtermittelkonzentrate für die Tierhaltung, die in üblicher Weise neben den Wirkstoffen auch Vitamine und/oder Mineralsalze enthalten können oder pharmazeutische Zubereitungen.

Die Erfindung betrifft bevorzugt antibakteriell wirksame Mittel, die Verbindungen der Formel (VIII) enthalten. Die Erfindung betrifft besonders bevorzugt solche antibakteriell wirksamen Mittel, die Verbindungen der Formel (IX) oder deren Alkali- oder Erdalkalisalze enthalten.

Die erfindungsgemäßen pharmazeutischen Zubereitungen enthalten neben den neuen erfindungsgemäßen Verbindungen in üblicher Weise nicht toxische, inerte pharmazeutisch geeignete Trägerstoffe. Solche pharmazeutisch geeigneten Trägerstoffe sind beispielsweise Füll- und Streckmittel, Bindemittel, Feuchthaltemittel, Lösungsverzögerer, Resorptionsbeschleuniger, Netzmittel, Adsorbtionsmittel oder Gleitmittel, die feste, halbfeste oder flüssige Konsistenz haben können. Solche pharmazeutisch geeignete Trägerstoffe sind dem Fachmann bekannt.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotionen, Puder und Sprays genannt. Die Herstellung dieser Zubereitungen geschieht nach bekannten Methoden in üblicher Weise, beispielsweise durch Mischen des neuen erfindungsgemäßen Wirkstoffes mit den üblichen Träger- und Zusatzstoffen. Der Wirkstoff soll in den aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Das zum Stand der Technik gehörende Verfahren zur Herstellung von 4-Chinolon-3-carbonsäurederivaten durch Reaktion von aromatischen Aminen mit Alkoxymethylen-Malonsäurediäthylester ergibt nur bei nichtsubstituierten aromatischen Aminen eindeutige Reaktionsprodukte. Bei substituierten aromatischen Aminen werden häufig Isomerengemische erhalten (J. Het. Chem. 12, 557 [1975]).

Der Vorteil des erfindungsgemäßen Verfahrens ist es, daß in einer einfach durchzuführenden, sogenannten Eintopf-Reaktion, nur einheitliche Verbindungen entstehen.

Es ist als ausgesprochen überraschend zu bezeichnen, daß die Enamine der Formel (I) durch die o-Halogen-(hetero)aroyl-halogenide der Formel (II) in der ersten Reaktionsstufe des erfindungsgemäßen Verfahrens an dem zum Stickstoff in $\beta$-Stellung befindlichen Kohlenstoff acyliert werden, während aus Chem. Ber., 50, 65 (1917), bekannt ist, daß Benzoylchloride, die in ortho-Stellung durch Brom, Nitro oder Acetoxy substituiert sind, nur unter Stickstoff-Acylierung reagieren. Weiterhin ist es überraschend, daß die Zwischenprodukte der ersten Reaktionsstufe des erfindungsgemäßen Verfahrens der Formel (VI) trotz der durch die Doppelbindung möglichen cis-trans-Isomerie in eindeutiger Weise zu den erfindungsgemäßen Endprodukten der Formel (VII) weiterreagieren.

Die Bereitstellung neuer Bakterizide zur Bekämpfung von Bakterien, die gegen bekannte Bakterizide resistent geworden sind, ist eine Bereicherung des Standes der Technik.

## Beispiel 1

Eine Lösung von 43,9 g 2-Chlor-6-methyl-nicotinsäurechlorid in 120 ml absolutem Dioxan wird unter Eiskühlung nacheinander tropfenweise mit 33 g $\beta$-Methylaminocrotonsäureäthylester und 23,5 g Triäthylamin versetzt. Man rührt 2 Stunden bei 25° C, tropft 72 g 1,8-Diazabicylo-[5.4.0]-undec-7-en(DBU) zu und erhitzt 5 Stunden unter Rückfluß. Dann wird das Lösungsmittel im Vakuum abdestilliert, und der Rückstand in einem Chloroform-Wasser-Gemisch aufgenommen. Die Chloroform-Phase wird über Natriumsulfat getrocknet und das Chloroform im Vakuum abdestilliert. Durch Umkristallisation aus Toluol/Cyclohexan erhält man 32,6 g (54% der theoretischen Ausbeute) 1,2,7-Trimethyl-4-oxo-1,8-naphthyridin-3-carbonsäureäthylester vom Schmelzpunkt 122 bis 123° C.

## Esterverseifung

26 g 1,2,7-Trimethyl-4-oxo-1,8-naphthyridin-3-carbonsäureäthylester werden mit einer Lösung von 6,2 g Kaliumhydroxid in 100 ml Wasser und 100 ml Äthanol etwa 4 Stunden unter Rückfluß zum Sieden erhitzt. Der Äthylalkohol wird im Vakuum abdestilliert, die wäßrige Lösung, gegebenenfalls unter Zusatz von weiterem Wasser, filtriert und das Filtrat unter Eiskühlung mit 10%iger Salzsäure bis zu einem pH-Wert von 1 bis 2 angesäuert. Der entstehende Niederschlag wird abgesaugt, mit Wasser gewaschen und im Vakuum bei etwa 60 bis 80° C getrocknet. Die erhaltene freie Carbonsäure kann aus Äthylalkohol umkristallisiert werden. Ausbeute: 19 g; Schmelzpunkt 224° C.

## Beispiele 2 bis 18

Analog der Arbeitsweise im Beispiel 1 wurden die Ester und Carbonsäuren der Beispiele 2 bis 18 erhalten. Sie sind in der Tabelle 1 zusammengestellt. Die Bezifferung der Reste A, B, D, E, $R^1$, $R^2$ und $R^3$ bezieht sich auf die Formel (I) der Beschreibung.

Tabelle 1

| Bei-spiel Nr. | A | B | D | E | $R^1$ | $R^2$ | $R^3$ | Fp (°C) (Ester) | Fp (°C) (Säure) |
|---|---|---|---|---|---|---|---|---|---|
| 2 | CH | CH | $C-NO_2$ | CH | 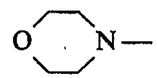 | $CH_3$ | $-COOC_2H_5$ | 211 | 210 (Z)*) |
| 3 | CH | CH | $C-NO_2$ | CH | $CH_3$ | $CH_3$ | $-COOC_2H_5$ | 228 | 290 (Z) |
| 4 | CH | CH | $C-NO_2$ | CH | $(CH_3)_2N-$ | $CH_3$ | $-COOCH_3$ | 203 | 277 (Z) |
| 5 | CH | $C-Cl$ | $C-NO_2$ | CH | $(CH_3)_2N-$ | $CH_3$ | $-COOCH_3$ | 193 | – |
| 6 | $C-NO_2$ | CH | CH | CH | $CH_3$ | $CH_3$ | $-COOCH_3$ | 238 | 285 (Z) |
| 7 | CH | CH | $C-NO_2$ | CH | $CH_3$ | $CH_3$ | $-CN$ | 216 | – |
| 8 | N | $C-SC_2H_5$ | N | CH | 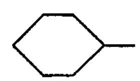 | H | $-COOCH_3$ | 147 | 229 (Z) |
| 9 | CH | CH | $C-NO_2$ | CH | $C_2H_5$ | $CH_3$ | $-COOCH_3$ | 205 | 242 (Z) |
| 10 | CH | CH | $C-NO_2$ | CH | $CH_3O$ 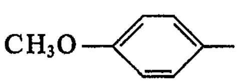 | $CH_3$ | $COOC_2H_5$ | 212 | 317 (Z) |
| 11 | CH | CH | $C-NO_2$ | CH | 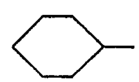 | $CH_3$ | $COOCH_3$ | 211 | – |
| 12 | N | $C-CH_3$ | CH | CH | $-(CH_2)_2\overset{H}{N}-COCH_3$ | $CH_3$ | $COOCH_3$ | 204 | 285 (Z) |
| 13 | N | $C-CH_3$ | CH | CH | $CH_3$ | $CH_3$ | $COOCH_3$ | 162 | 230 (Z) |

Fortsetzung

| Bei-spiel Nr. | A | B | D | E | $R^1$ | $R^2$ | $R^3$ | Fp (°C) (Ester) | Fp (°C) (Säure) |
|---|---|---|---|---|---|---|---|---|---|
| 14 | CH | CH | $C-NO_2$ | CH | 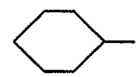 | $CH_3$ | $COOC_2H_5$ | 210 | 204 |
| 15 | N | $C-CH_3$ | CH | CH | $C_2H_5$ | $CH_3$ | $COOCH_3$ | 160 | 226 (Z) |
| 16 | N | $C-SC_2H_5$ | N | CH | $CH_3$ | $CH_3$ | $COOCH_3$ | 184 | 252 (Z) |
| 17 | CH | $C-SC_4H_9-n$ | $C-NO_2$ | CH | $(CH_3)_2-N$ | $CH_3$ | $COOCH_3$ | 204 | 184 |
| 18 | CH | CH | $C-NO_2$ | CH | $C_6H_5$ | $CH_3$ | $COOC_2H_5$ | 220 | – |
| 19 | N | CH | CH | CH | 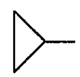 | H | $COOCH_3$ | 190–192 | |
| 20 | N | $C-CH(CH_3)_2$ | CH | CH | 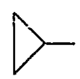 | H | $COOCH_3$ | 181–186 | |

*) (Z) bedeutet: Schmelzpunkt unter (teilweiser) Zersetzung.

Beispiel 21

Zu einer Lösung von 19 g 2-Chlor-6-methyl-nicotinsäurechlorid in 70 ml absolutem Dioxan tropft man unter Eiskühlung und Rühren zunächst 14,1 g β-Cyclopropylaminoacrylsäuremethylester und anschließend 10,4 g Triäthylamin. Man rührt 4 Stunden bei Raumtemperatur und 0,5 Stunden bei 40° C, versetzt unter Eiskühlung mit 31 g 1,8-Diazabicyclo-[5.4.0]-undec-7-en(DBU) und erhitzt 6 Stunden unter Rückfluß. Das Lösungsmittel wird anschließend im Vakuum abdestilliert und der Rückstand in einem Chloroform-Wasser-Gemisch aufgenommen. Die Chloroform-Phase wird mit Natriumsulfat getrocknet und das Chloroform abdestilliert. Nach Umkristallisation aus Acetonitril erhält man 8,5 g 1-Cyclopropyl-7-methyl-4-oxo-1,8-naphthyridin-3-carbonsäuremethylester vom Schmelzpunkt 204 bis 205° C. Die durch Verseifung analog Beispiel 1 erhaltene entsprechende Carbonsäure schmilzt bei 229 bis 230° C (unter Zersetzung).

Beispiel 22

Eine Lösung von 38 g 2-Chlor-6-methyl-nicotinsäurechlorid in 120 ml absolutem Dioxan wird unter Eiskühlung und Rühren tropfenweise mit 33,8 g β-Cyclopentylaminoacrylsäuremethylester in 20 ml Dioxan und dann tropfenweise mit 20,4 g Triäthylamin versetzt, wobei eine Temperatur von 10 bis 15° C eingehalten wird. Dann wird 4 Stunden bei Raumtemperatur und 0,5 Stunden bei etwa 40° C gerührt, nach Abkühlen auf 20° C mit 63 g DBU versetzt und 7 Stunden unter Rückfluß erhitzt. Anschließend wird das Dioxan im Vakuum abdestilliert und der Rückstand in Chloroform aufgenommen und mit Wasser gewaschen. Nachdem das Chloroform abdestilliert worden ist, wird der rohe Ester direkt mit 80 ml Äthylalkohol und einer Lösung von 17 g Kaliumhydroxid in 80 ml Wasser 4 Stunden unter Rückfluß erhitzt. Der Äthylalkohol wird im Vakuum abdestilliert und das Kaliumsalz der entstandenen Carbonsäure in Wasser gelöst, filtriert und die Carbonsäure mit 10%iger Salzsäure unter Eiskühlung ausgefällt. Der abfiltrierte und getrocknete Niederschlag wird aus Äthylalkohol umkristallisiert. Man erhält 38,5 g 1-Cyclopentyl-7-methyl-4-oxo-1,8-naphthyridin-3-carbonsäure vom Schmelzpunkt 236 bis 237° C (unter Zersetzung).

Beispiele 23 bis 31

Analog der in Beispiel 20 angegebenen Arbeitsweise wird bei den Beispielen 21 bis 29 gearbeitet. Die Ergebnisse sind in der Tabelle 2 zusammengestellt. Die Symbole A bis $R^3$ beziehen sich wie bei der Tabelle 1 auf die Formel (I).

Tabelle 2

| Beispiel Nr. | A | B | D | E | $R^1$ | $R^2$ | $R^3$ verseift zu | Fp (°C) |
|---|---|---|---|---|---|---|---|---|
| 23 | N | $C—CH_3$ | CH | CH | 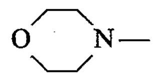 | $CH_3$ | COOH | 295 (Z)*) |
| 24 | N | $C—CH_3$ | CH | CH | 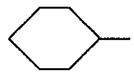 | H | COOH | 272 (Z) |
| 25 | N | $C—CH_3$ | CH | CH | $(CH_3)_3C—$ | H | COOH | 291 (Z) |
| 26 | N | $C—CH_3$ | CH | CH | $—(CH_2)_2\overset{H}{N}—COOC_2H_5$ | H | COOH | 199 (Z) |
| 27 | N | $C—CH_3$ | CH | CH | 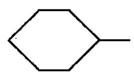 | $CH_3$ | COOH | 213 (Z) |
| 28 | N | $C—CH_3$ | CH | CH | $n\text{-}C_3H_5—$ | H | COOH | 208 (Z) |
| 29 | N | $C—CH_3$ | CH | CH | $C_2H_5$ | H | COOH | 227 (Z) |
| 30 | N | $C—CH_3$ | CH | CH | $CH_3$ | $CH_3$ | COOH | 230 (Z) |
| 31 | N | $C—SC_2H_5$ | N | CH | 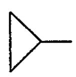 | H | COOH | 215 (Z) |

*) (Z) bedeutet: Schmelzpunkt unter (teilweiser) Zersetzung.

## Beispiel 32

65,4 g 2-Chlor-5-nitro-benzoylchlorid werden in 250 ml absolutem Dioxan unter Eiskühlung und Rühren portionsweise mit 77,5 g $\beta$-Morpholinylamino-crotonsäureanilid und dann tropfenweise mit 24 g Pyridin versetzt. Man rührt 4 Stunden bei Raumtemperatur und 2 Stunden bei 50 bis 60° C, destilliert das Dioxan im Vakuum ab und nimmt das Reaktionsgemisch in Wasser auf. Der entstandene Niederschlag wird abgesaugt und nach dem Trocknen aus Essigester/Acetonitril umkristallisiert. Die gelben Kristalle des $\alpha$-(2-Chlor-5-nitro-benzoyl)-$\beta$-morpholinyl-amino-crotonsäureanilids schmelzen bei 190 bis 191° C. Ausbeute: 48 g.

19,3 g Anilid werden in 100 ml Dioxan mit 7 g DBU 6 Stunden unter Rückfluß erhitzt. Das Dioxan wird anschließend im Vakuum abdestilliert und der Rückstand in Wasser aufgenommen. Der Niederschlag wird abgesaugt und mit Wasser gewaschen. Nach dem Trocknen wird aus Dimethylformamid/Äthanol umkristallisiert, und man erhält 14 g des 2-Methyl-1-morpholinyl-6-nitro-4-chinolon-3-carbonsäureanilids vom Schmelzpunkt 276° C (unter Zersetzung).

## Beispiel 33

Eine Lösung von 191,1 g 2-Chlor-5-nitro-benzoylchlorid in 500 ml wasserfreiem Dioxan wird unter Eiskühlung und Rühren tropfenweise mit 138 g $\beta$-(2,2-Dimethylhydrazino)-crotonsäuremethylester versetzt. Dann tropft man bei etwa 10 bis 15° C 88,2 g Triäthylamin zu, rührt eine Stunde bei Raumtemperatur und 2,5 Stunden bei 50 bis 60° C. Das Lösungsmittel wird im Vakuum abdestilliert und der Rückstand in einem Chloroform-Wasser-Gemisch aufgenommen. Die Chloroform-Phase wird über Natriumsulfat getrocknet. Anschließend wird das Chloroform im Vakuum abdestilliert. Nach Umkristallisation aus Essigsäureäthylester erhält man 215,5 g $\alpha$-(2-chlor-5-nitro-benzoyl)-$\beta$-(2,2-dimethylhydrazino)-crotonsäuremethylester. Schmelzpunkt 142 bis 143° C.

34 g des beschriebenen Esters werden mit einer Lösung von 5,7 g Kaliumhydroxid in 150 ml Äthylalkohol versetzt. Dann wird 0,5 Stunden auf 40° C und 4 Stunden unter Rückfluß erhitzt. Nach dem Aufarbeiten wie in der ersten Reaktionsstufe erhält man 19,8 g hellgelbe Kristalle des

1-Dimethylamino-2-methyl-6-nitro-4-chinolon-3-carbonsäuremethylesters. Schmelzpunkt 201 bis 202° C.

Beispiel 34

Zu einer Lösung von 64,2 g β-Morpholinylamino-crotonsäureäthylester und 24 g Pyridin in 120 ml absolutem Dioxan tropft man unter Eiskühlung und Rühren eine Lösung von 66 g 2-Chlor-5-nitro-benzoylchlorid in 30 ml Dioxan. Man rührt eine Stunde bei Raumtemperatur und eine Stunde bei etwa 40° C. Es wird wie in Beispiel 31 aufgearbeitet. Nach Umkristallisation aus Methanol erhält man 82,3 g α-(2-Chlor-5-nitro-benzoyl)-β-morpholinylamino-crotonsäureäthylester. Schmelzpunkt 137 bis 138° C.

a) Cyclisierung mit DBU als Base
39,7 g des beschriebenen acyclischen Esters werden in 100 ml absolutem Dioxan mit 16 g DBU 6 Stunden unter Rückfluß erhitzt. Das Dioxan wird im Vakuum abdestilliert und der Rückstand wie in Beispiel 31 aufgearbeitet und aus Äthanol umkristallisiert. Man erhält 16,5 g 1-Morpholinyl-amino-2-methyl-6-nitro-4-chinolon-3-carbonsäureäthylester vom Schmelzpunkt 210 bis 211° C.

b) Cyclisierung mit Natriumäthylat
Eine Lösung von 1,54 g metallischem Natrium in 150 ml absolutem Äthanol versetzt man unter Eiskühlung und Rühren portionsweise mit 26,5 g des oben beschriebenen acyclischen Esters und erhitzt anschließend 4 Stunden unter Rückfluß. Es wird wie im Beispiel 31 aufgearbeitet und aus Äthanol/Acetonitril umkristallisiert. Man erhält 18,6 g 1-Morpholinylamino-2-methyl-6-nitro-4-chinolon-3-carbonsäureäthylester vom Schmelzpunkt 209 bis 210° C.

c) Cyclisierung mit Kaliumhydroxid/Äthylalkohol
3,8 g Kaliumhydroxid in 150 ml Äthanol werden rasch mit 26,5 g des oben beschriebenen acyclischen Esters versetzt. Man erhitzt 4 Stunden unter Rückfluß und arbeitet wie zuvor beschrieben auf. Nach der Umkristallisation aus Äthylalkohol/Acetonitril erhält man 22 g des bereits unter a) und b) beschriebenen 4-Chinolonesters vom Schmelzpunkt 209 bis 211° C.

Die durch Verseifung erhältliche 1-Morpholinylamino-2-methyl-6-nitro-4-chinolon-3-carbonsäure schmilzt bei 265° C (unter Zersetzung).

**Patentansprüche**

1. Verfahren zur Herstellung von 4-Pyridon-3-carbonsäuren und/oder deren Derivaten, dadurch gekennzeichnet, daß man Enamine der Formel

$$\begin{array}{l} R^2-C=CH-R^3 \\ \quad\;\; | \\ R^1-NH \end{array} \qquad (I)$$

in der

$R^1$ gesättigtes oder ungesättigtes, geradkettiges oder verzweigtes Alkyl mit 1–6 Kohlenstoffatomen, gesättigtes oder ungesättigtes, gegebenenfalls durch Methyl- oder Äthylgruppen substituiertes Cycloalkyl mit 3–7 Kohlenstoffatomen, Aralkyl mit 1–4 Kohlenstoffatomen im aliphatischen Teil und 6–10 Kohlenstoffatomen im aromatischen Teil, Aryl mit 6–10 Kohlenstoffatomen oder eine Aminogruppe $-NR^4R^5$ bedeutet, in der $R^4$ und $R^5$ gleich oder verschieden sein können und für geradkettiges oder verzweigtes $C_1-C_4$-Alkyl stehen oder gemeinsam mit dem Stickstoffatom, das sie substituieren und gegebenenfalls einem weiteren Heteroatom einen 5- bis 7gliedrigen Ring bilden können,

$R^2$ Wasserstoff, gesättigtes oder ungesättigtes, geradkettiges oder verzweigtes Alkyl mit 1–6 Kohlenstoffatomen, Aralkyl mit 1–4 Kohlenstoffatomen im aliphatischen Teil und 6–10 Kohlenstoffatomen im aromatischen Teil, oder Aryl mit 6–10 Kohlenstoffatomen bedeutet und

$R^3$ eine Nitrilgruppe, eine Estergruppe $-COOR^6$ oder eine Säureamidgruppe $-CONR^7R^8$, wobei $R^6$, $R^7$ und $R^8$ für Wasserstoff oder $C_1-C_4$-Alkyl stehen, $R^8$ außerdem gegebenenfalls substituiertes Phenyl sein kann, bedeutet,

mit o-Halogen-(hetero)aryl-carbonsäurehalogeniden der Formel

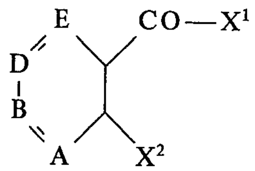

(II)

in der

bis zu 3 der Symbole A, B, D und E ein Stickstoffatom sein können und die jeweils restlichen der Symbole A, B, D und E ein gegebenenfalls durch $C_1-C_6$-Alkyl, $C_1-C_4$-Alkoxy, $C_1-C_6$-Alkylmerkapto, Trifluormethyl, Halogen, Cyano, durch $C_1-C_4$-Alkyl verestertes Carboxyl, Benzyl oder Phenyl, die jeweils durch $C_1-C_3$-Alkyl, Nitro oder Halogen substituiert sein können, oder durch Carbalkoxy substituiertes Amino substituiertes Kohlenstoffatom bedeuten, und
$X^1$ und $X^2$ gleiches oder verschiedenes Halogen darstellen,

in einer ersten Reaktionsstufe in einem wasserfreien, aprotischen Lösungsmittel in Gegenwart einer Base bei 0 bis 80° C und in einer zweiten Reaktionsstufe in Gegenwart einer Base bei etwa 80 bis 250° C umsetzt und gegebenenfalls die Gruppe $R^3$ in an sich bekannter Weise in die Carboxylgruppe und gegebenenfalls diese in ihre Salze überführt.

2. Verfahren nach Anspruch 1, dadurch gekennzeichnet, daß man als aprotisches Lösungsmittel Dioxan verwendet.

3. Verfahren nach Anspruch 1 und 2, dadurch gekennzeichnet, daß man in der ersten Reaktionsstufe bei etwa 10 bis 60° C und in der zweiten Reaktionsstufe bei etwa 100 bis 150° C arbeitet.

4. Verfahren nach Anspruch 1 bis 3, dadurch gekennzeichnet, daß man in der ersten Reaktionsstufe Triäthylamin als Base und in der zweiten Reaktionsstufe 1,8-Diazobicyclo-[5,4,0]-undec-7-en als Base einsetzt.

5. Verfahren nach Anspruch 1 bis 4, dadurch gekennzeichnet, daß man Enamine der Formel

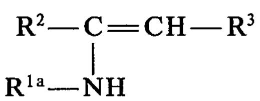

(III)

in der

$R^{1a}$ tert.-Alkyl, $C_3-C_7$-Cycloalkyl, die Dialkylgruppe $NR^4-R^5$, worin $R^4$ und $R^5$ $C_1-C_2$-Alkyl bedeutet, oder N-Morpholinyl darstellt, und
$R^2$ für Wasserstoff, $C_1-C_4$-Alkyl oder gegebenenfalls substituiertes Benzyl oder Phenyl steht, und
$R^3$ die oben angegebene Bedeutung besitzt,

einsetzt.

6. Verfahren nach Anspruch 1 bis 4, dadurch gekennzeichnet, daß man Enamine der Formel

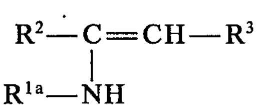

(IV)

in der

$R^{1a}$ tert.-Butyl, Cyclopropyl, Cyclohexyl, Dimethylamino oder N-Morpholinyl darstellt und
$R^2$ für Wasserstoff oder $C_1-C_4$-Alkyl steht und
$R^3$ die oben angegebene Bedeutung besitzt,

einsetzt.

7. Verfahren nach Anspruch 1 bis 6, dadurch gekennzeichnet, daß man o-Chlor-(hetero)aryl-carbonsäurechloride der Formel

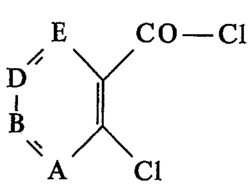

(V)

in der die Symbole A, B, D und E die oben genannte Bedeutung haben, der gegebenenfalls vorhandene Heterocyclus jedoch höchstens zwei Stickstoffatome enthält, einsetzt.

8. 4-Pyridon-3-carbonsäuren der Formel

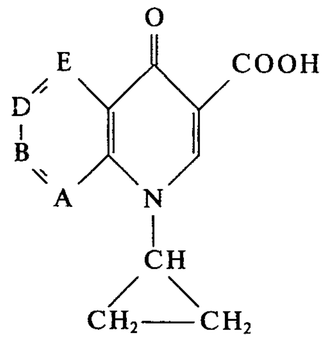

(IX)

in der A, B, D und E die oben angegebene Bedeutung haben, und deren pharmazeutisch verwendbaren Salze.

9. Arzneimittel, gekennzeichnet durch einen Gehalt an mindestens einer Verbindung der in Anspruch 8 angegebenen Verbindungen und üblichen Hilfs- und Trägerstoffen.

**Claims**

1. Process for the preparation of 4-pyridone-3-carboxylic acids and/or derivatives thereof, characterised in that enamines of the formula

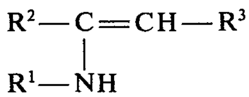

(I)

in which

$R^1$ denotes saturated or unsaturated, straight-chain or branched alkyl with 1–6 carbon atoms, saturated or unsaturated cycloalkyl with 3–7 carbon atoms which is optionally substituted by methyl or ethyl groups, aralkyl with 1–4 carbon atoms in the aliphatic part and 6–10 carbon atoms in the aromatic part, aryl with 6–10 carbon atoms or an amino group $-NR^4R^5$, in which $R^4$ and $R^5$ can be identical or different and represent straight-chain or branched $C_1-C_4$-alkyl or together with the nitrogen atom which they substitute and optionally a further hetero-atom can form a 5-membered to 7membered ring,

$R^2$ denotes hydrogen, saturated or unsaturated, straight-chain or branched alkyl with 1–6 carbon atoms, aralkyl with 1–4 carbon atoms in the aliphatic part and 6–10 carbon atoms in the aromatic part, or aryl with 6–10 carbon atoms and

$R^3$ denotes a nitrile group, an ester group $-COOR^6$ or an acid amide group $-CONR^7R^8$, wherein $R^6$, $R^7$ and $R^8$ represent hydrogen or $C_1-C_4$-alkyl, and $R^8$ can furthermore be optionally substituted phenyl,

are reacted with o-halogeno-(hetero-)aryl-carboxylic acid halides of the formula

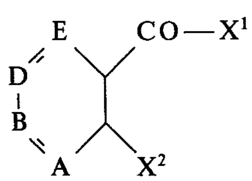

(II)

in which

up to 3 of the symbols A, B, D and E can be a nitrogen atom and those of the symbols A, B, D and E remaining in each case denote a carbon atom which is optionally substituted by $C_1-C_6$-alkyl, $C_1-C_4$-alkoxy, $C_1-C_6$-alkylmercapto, trifluoromethyl, halogen, cyano, carboxyl which is esterified by $C_1-C_4$-alkyl, benzyl or phenyl which can in each case be substituted by $C_1-C_3$-alkyl, nitro or halogen, or amino substituted by carbalkoxy, and

$X^1$ and $X^2$ represent identical or different halogen,

in a first reaction stage at 0 to 80° C in an anhydrous, aprotic solvent and in the presence of a base, and in a second reaction stage at about 80 to 250° C in the presence of a base, if appropriate the group $R^3$ is converted into the carboxyl group in a manner which is in itself known, and if appropriate this carboxyl group is converted into its salts.

2. Process according to Claim 1, characterised in that dioxane is used as the aprotic solvent.

3. Process according to Claim 1 and 2, characterised in that the first reaction stage is carried out at about 10 to 60° C and the second reaction stage is carried out at about 100 to 150° C.

4. Process according to Claim 1 to 3, characterised in that triethylamine is employed as the base in the first reaction stage and 1,8-diazobicyclo-[5.4.0]-undec-7-ene is employed as the base in the second reaction stage.

5. Process according to Claim 1 to 4, characterised in that enamines of the formula

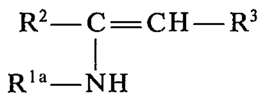

(III)

in which

$R^{1a}$ represents tert.-alkyl, $C_3-C_7$-cycloalkyl, the dialkyl group $-NR^4-R^5$, wherein $R^4$ and $R^5$ denote $C_1-C_2$-alkyl, or N-morpholinyl,
$R^2$ represents hydrogen, $C_1-C_4$-alkyl or optionally substituted benzyl or phenyl and
$R^3$ has the meaning indicated above,

are employed.

6. Process according to Claim 1 to 4, characterised in that enamines of the formula

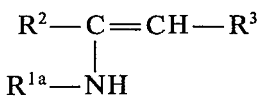

(IV)

in which

$R^{1a}$ represents tert.-butyl, cyclopropyl, cyclohexyl, dimethylamino or N-morpholinyl,
$R^2$ represents hydrogen or $C_1-C_4$-alkyl and
$R^3$ has the meaning indicated above,

are employed.

7. Process according to Claim 1 to 6, characterised in that o-chloro-(hetero-)aryl-carboxylic acid chlorides of the formula

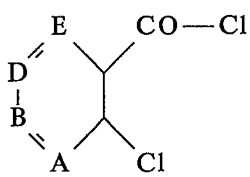

(V)

in which the symbols A, B, D and E have the abovementioned meaning, but the heterocyclic radical optionally present contains at most two nitrogen atoms, are employed.

8. 4-Pyridone-3-carboxylic acids of the formula

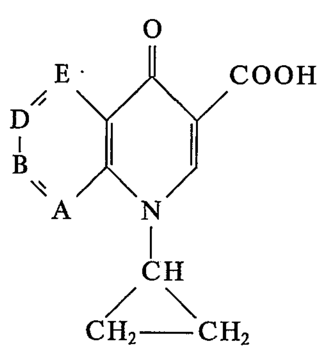

(IX)

in which A, B, D and E have the meaning indicated above, and pharmaceutically usable salts thereof.

9. Medicaments, characterised in that they contain at least one compound of the compounds indicated in Claim 8 and customary auxiliaries and excipients.

**Revendications**

1. Procédé de préparation d'acides 4-pyridone-3-carboxyliques et/ou de leurs dérivés, caractérisé en ce qu'on fait réagir des énamines de formule:

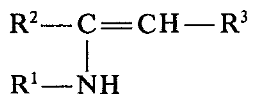

(I)

dans laquelle

$R^1$ représente un groupe alkyle saturé ou insaturé, à chaîne droite ou ramifiée et contenant 1 à 6 atomes de carbone, un groupe cycloalkyle saturé ou insaturé, contenant 3 à 7 atomes de carbone et éventuellement substitué par un groupe méthyle ou éthyle, un groupe aralkyle contenant 1 à 4 atomes de carbone dans la fraction aliphatique et 6 à 10 atomes de carbone dans la fraction aromatique, un groupe aryle contenant 6 à 10 atomes de carbone ou un groupe amino $-NR^4R^5$ où $R^4$ et $R^5$ peuvent être identiques ou différents et représentent chacun un groupe alkyle à chaîne droite ou ramifiée et contenant 1 à 4 atomes de carbone ou, ensemble avec l'atome d'azote qu'ils substituent, et éventuellement un autre hétéro-atome, ils peuvent former un noyau pentagonal à heptagonal,

$R^2$ représente un atome d'hydrogène, un groupe alkyle saturé ou insaturé, à chaîne droite ou ramifiée et contenant 1 à 6 atomes de carbone, un groupe aralkyle contenant 1 à 4 atomes de carbone dans la fraction aliphatique et 6 à 10 atomes de carbone dans la fraction aromatique, ou un groupe aryle contenant 6 à 10 atomes de carbone, et

$R^3$ représente un groupe nitrile, un groupe ester $-COOR^6$ ou un groupe d'amide d'acide $-CONR^7R^8$, $R^6$, $R^7$ et $R^8$ représentant chacun un atome d'hydrogène ou un groupe alkyle contenant 1 à 4 atomes de carbone, $R^8$ pouvant, en outre, représenter un groupe phényle éventuellement substitué,

avec des halogénures d'acides o-halogéno-(hétéro)-aryl-carboxyliques de formule:

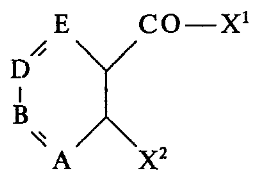

(II)

dans laquelle

jusqu'à trois des symboles A, B, D et E peuvent être chacun un atome d'azote, tandis que les autres symboles A, B, D et E représentent chacun un atome de carbone éventuellement substitué par un groupe alkyle en $C_1-C_6$, par un groupe alcoxy en $C_1-C_4$, par un groupe alkyl(en $C_1-C_6$)mercapto, par un groupe trifluorométhyle, par un atome d'halogène, par un groupe cyano, par un groupe phényle, benzyle ou carboxy estérifié par un groupe alkyle en $C_1-C_4$ et pouvant être substitué chacun par un groupe alkyle en $C_1-C_3$, par un groupe nitro ou par un atome d'halogène, ou encore un atome de carbone éventuellement substitué par un groupe amino substitué par un groupe carbalcoxy, et

$X^1$ et $X^2$ représentent chacun un atome d'halogène identique ou différent,

dans une première étape réactionnelle dans un solvant aprotique anhydre, en présence d'une base à une température de 0 à 80° C et, dans une deuxième étape réactionnelle, en présence d'une base à une température d'environ 80 à 250° C, et on transforme éventuellement le groupe $R^3$, de façon connue en soi, en un groupe carboxy et éventuellement ce dernier, en ses sels.

2. Procédé suivant la revendication 1, caractérisé en ce que, comme solvant aprotique, on utilise le dioxanne.

3. Procédé suivant les revendications 1 et 2, caractérisé en ce que, lors de la première étape réactionnelle, on travaille à une température d'environ 10 à 60° C tandis que, lors de la deuxième étape réactionnelle, on travaille à une température d'environ 100 à 150° C.

4. Procédé suivant les revendications 1 à 3, caractérisé en ce que, lors de la première étape réactionnelle, on utilise la triéthylamine comme base tandis que, lors de la deuxième étape réactionnelle, on utilise le 1,8-diazabicyclo-[5,4,0]-undéc-7-ène comme base.

5. Procédé suivant les revendications 1 à 4, caractérisé en ce qu'on utilise des énamines de formule:

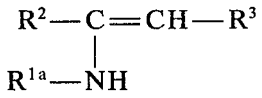

(III)

dans laquelle

$R^{1a}$ représente un groupe tert-alkyle, un groupe cycloalkyle en $C_3-C_7$, le groupe dialkyle $-NR^4-R^5$ dans lequel $R^4$ et $R^5$ représentent chacun un groupe alkyle en $C_1-C_2$, ou le groupe N-morpholinyle, et

$R^2$ représente un atome d'hydrogène, un groupe alkyle en $C_1-C_4$ ou un groupe phényle ou benzyle éventuellement substitué, et

$R^3$ a la signification indiquée ci-dessus.

6. Procédé suivant les revendications 1 à 4, caractérisé en ce qu'on utilise des énamines de formule:

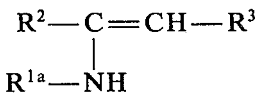

(IV)

dans laquelle

$R^{1a}$ représente un groupe tert-butyle, cyclopropyle, cyclohexyle, diméthylamino ou N-morpholinyle,

$R^2$ représente un atome d'hydrogène ou un groupe alkyle en $C_1-C_4$ et

$R^3$ a la signification indiquée ci-dessus.

7. Procédé suivant les revendications 1 à 6, caractérisé en ce qu'on utilise des chlorures d'acides o-chloro-(hétéro)aryl-carboxyliques de formule:

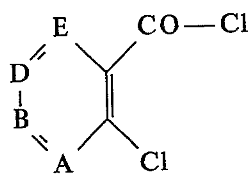

(V)

dans laquelle les symboles A, B, D et E ont les significations indiquées ci-dessus, cependant que l'hétérocycle éventuellement présent contient, au maximum, deux atomes d'azote.

8. Acides 4-pyridone-3-carboxyliques de formule:

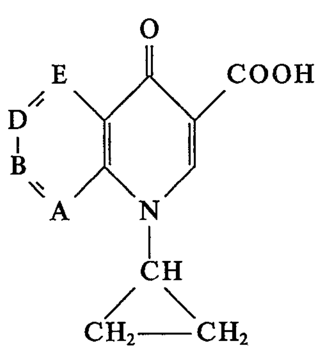

(IX)

dans laquelle A, B, D et E ont les significations indiquées ci-dessus, ainsi que leurs sels pharmaceutiquement utilisables.

9. Médicaments caractérisés en ce qu'ils contiennent au moins un composé choisi parmi ceux indiqués dans la revendication 8, ainsi que des substances auxiliaires et des substances supports habituelles.